Europäisches Patentamt

European Patent Office

Office européen des brevets

(19)

(11) Numéro de publication: **0 239 450**
**A1**

(12) **DEMANDE DE BREVET EUROPEEN**

(21) Numéro de dépôt: 87400428.6

(22) Date de dépôt: 26.02.87

(51) Int. Cl.⁴: **A 61 D 7/02**
C 12 M 3/00, A 61 B 17/42,
A 01 K 67/02

(30) Priorité: 26.02.86 FR 8602697

(43) Date de publication de la demande:
30.09.87 Bulletin 87/40

(84) Etats contractants désignés:
AT BE CH DE ES GB GR IT LI LU NL SE

(71) Demandeur: Cassou, Robert
Rue Clémenceau
F-61300 L'Aigle (FR)

Cassou, Bertrand
Saint Symphorien des Bruyeres
F-61300 L'Aigle (FR)

Cassou, Maurice
Rue Clémenceau
F-61300 L'Aigle (FR)

(72) Inventeur: Cassou, Robert
Rue Clémenceau
F-61300 L'Aigle (FR)

Cassou, Bertrand
Saint Symphorien des Bruyeres
F-61300 L'Aigle (FR)

Cassou, Maurice
Rue Clémenceau
F-61300 L'Aigle (FR)

(74) Mandataire: Rodhain, Claude et al
Cabinet Claude Rodhain 30, rue la Boétie
F-75008 Paris (FR)

(54) Appareillage et procédé de fécondation d'ovocytes in-vitro, et enceinte de fécondation et de culture cellulaire mise en oeuvre dans cet appareillage et ce procédé.

(57) L'invention concerne un appareillage et un procédé de fécondation in-vitro et de culture cellulaire, et également d'examen des ovocytes ainsi que des embryons obtenus. Elle concerne également des enceintes de fécondation et de culture particulièrement adaptées.

L'invention est caractérisée en ce que les enceintes (100) comportent des bassins (103) pour recevoir un ovocyte, des gamètes et des cellules, et sont placées sur un carrousel (1) disposé dans une couveuse à atmosphère conditionnée, de sorte que les enceintes passent, pas à pas, sous une loupe binoculaire tout en étant alimentées en une atmosphère gazeuse stérile, les bassins étant éclairés et examinés à travers des parois transparentes, les enceintes comportant par ailleurs une cavité recevant un liquide d'humidification.

FIG.1

## Description

"Appareillage et procédé de fécondation d'ovocytes in-vitro, et enceinte de fécondation et de culture cellulaire mise en oeuvre dans cet appareillage et ce procédé"

L'invention concerne un appareillage et un procédé de fécondation in-vitro utilisables aussi bien pour les humains que pour les espèces animales, notamment un appareillage et un procédé de fécondation in-vitro d'ovocytes, et d'examen des embryons obtenus. L'invention concerne également une enceinte de fécondation et de culture cellulaire, enceinte où sont placés les ovocytes à féconder et les spermatozoïdes.

Les conditions optimales de culture : milieu, ambiance gazeuse maintenant les pH, température, sont bien définies et doivent être scrupuleusement respectées.

On sait déjà dans ce domaine réaliser des ponctions folliculaires pour prélever des ovocytes au moyen d'une aiguille, et les transférer dans un tube à essais comportant un bouchon en silicone traversé par deux tuyaux, reliés l'un à une pompe, en passant par un vase de décantation,pour créer une dépression, l'autre, en passant par une valve en silicone,à l'aiguille de ponction de follicule reliée par ailleurs à un dispositif de lavage. Les ovocytes ainsi récupérés sont identifiés et lavés, puis, dans un second tube à essais, mis en présence de semence, c'est-à-dire de spermatozoïdes sélectionnés mobiles. Lorsque les ovocytes ont été fécondés par les spermatozoïdes, les embryons obtenus sont placés dans des boîtes connues sous le nom de boîtes de Pétri ou coupelles d'examen et de conditionnement, pour faciliter leur observation au moyen d'une loupe binoculaire sous une hotte à flux laminaire, et restent sous couveuse jusqu'à ce que ces embryons aient atteint le développement permettant leur transfert dans l'utérus d'une receveuse.

Plus précisément, la culture peut être effectuée soit en tube à essais muni d'un bouchon hermétique et permettant le maintien de l'atmosphère gazeuse apporpriée, mais l'observation des embryons nécessite plusieurs transferts dans une coupelle d'examen, soit dans les boîtes de Pétri elles-mêmes, ce qui permet une observation plus facile, mais ces boîtes de Pétri ou coupelles n'étant jamais hermétiques doivent être placées dans une couveuse sous atmosphère gazeuse.

Cette technique nécessite donc de nombreux transferts, en particulier, du tube à essais, permettant la fécondation, aux boîtes de Pétri, permettant l'observation, ce qui implique de multiples transvasements et manipulations et en conséquence des risques nombreux de pertes d'ovocytes et de contamination, ne serait-ce que par l'air ambiant, ainsi que des chocs mécaniques et des chocs thermiques dus aux changements de température des ovocytes et des embryons, lors de leur transvasement en dehors de la couveuse. De plus, on doit parfois utiliser de grandes quantités de gaz servant à maintenir une atmosphère stérile dans les conditions de température et de pression appropriées et il n'est de toute manière pas possible d'assurer simultanément un gazage correct et une observations facile. Egalement, le contrôle du pH et de la température du milieu liquide, dans lequel sont cultivés les ovocytes pour devenir des embryons, est difficile. En conséquence, le temps de manipulation passé par les utilisateurs est important, et le confort et la sécurité de ces manipulations sont au plus acceptables. Enfin, dans la mesure où les tubes à essais sont placés dans un bain-marie, il existe, à chacun de leur retrait du bain, de sérieux risques d'égouttage d'eau dans les couveuses.

L'invention a pour but de remédier à ces inconvénients et concerne à cet effet un appareillage de fécondation d'ovocytes in-vitro, du type comprenant des enceintes de fécondation comportant des bassins pour recevoir les ovocytes dans un milieu approprié ainsi que des spermatozoïdes en vue de la fécondation desdits ovocytes afin d'obtenir des embryons susceptibles d'être transférés dans une mère-receveuse, lorsqu'ils ont atteint un développement approprié, appareillage caractérisé en ce qu'il comporte un dispositif mobile, tel qu'un carrousel, ayant au moins un plateau mobile en rotation pas à pas et muni d'emplacements de positionnement destinés à recevoir chacun une enceinte de fécondation, éventuellement un capot transparent de fermeture entourant ledit dispositif mobile, un dispositif optique binoculaire de grossissement pour examiner successivement le contenu des bassins, par le dessus de ceux-ci et du capot de fermeture éventuel, lequel est muni d'une fenêtre en regard de l'optique de grossissement, et des moyens d'éclairage du contenu des bassins de fécondation.

Grâce d'une part au fait que les enceintes de fécondation elles-mêmes peuvent être fixées sur un plateau mobile, en vue de l'examen du contenu de leurs bassins, et d'autre part du fait de leur conception, il n'est nul besoin de transvaser les ovocytes ou embryons pour les examiner, ce qui limite le nombre d'ouvertures des enceintes, et les risques de pollution à condition que les enceintes possèdent un couvercle étanche et transparent, tout en faisant gagner un temps important à l'opérateur et en lui assurant un grand confort de manipulation en vue d'obtenir les meilleurs taux de fécondation ou de culture.

Selon une caractéristique de l'invention, l'appareillage comporte des moyens d'alimentation des enceintes disposées sur le dispositif mobile, en atmosphère gazeuse. Seul l'intérieur des enceintes étant empli de gaz, l'économie de gaz, par rapport aux installations classiques avec hotte à flux laminaire,est donc importante.

L'invention concerne également un procédé de fécondation d'ovocytes in-vitro dans lequel on prélève les ovocytes par ponction folliculaire au moyen d'une aiguille, procédé caractérisé en ce que l'on identifie les ovocytes aptes à être fécondés dans de bonnes conditions, on transfère chaque ovocyte dans un bassin respectif d'une enceinte de fécondation, on introduit dans les bassins un milieu liquide adapté à la fécondation, on place les spermatozoïdes, préalablement filtrés donc vivants dans les bassins identifiés, on ferme de manière étanche le couvercle de l'enceinte de fécondation, on ouvre alors une arrivée de gaz stérile et réchauffé éventuellement par barbotage pour éviter les chocs thermiques, et on introduit dans l'enceinte le gaz stérile qui chasse l'air résiduel, puis après écoulement d'un certain laps de temps, sans ouvrir l'enceinte, on examine le contenu des bassins au moyen d'un dispositif optique de grossissement.

Grâce au fait que l'on transfère les ovocytes dans un bassin d'une enceinte de fécondation et que l'on introduit dans ce bassin le milieu de fécondation compatible avec l'ovocyte, puis celui de l'embryon (jusqu'au transfert dans une mère porteuse), le dosage du volume de milieu peut n'être effectué qu'une seule fois, à l'aide d'une seringue stérile.

L'invention concerne aussi une enceinte de fécondation d'ovocytes pour appareillage de fécondation d'ovocytes in-vritro, caractérisée en ce qu'elle est est étanche et au moins partiellement transparente et en ce qu'elle comporte des bassins excentrés à paroi inclinée convergeant vers le bas et présentant un fond excentré incurvé à concavité tournée vers le bas et au moins une zone intermédiaire plane et horizontale, ainsi qu'au moins un ajutage d'entrée et de sortie de gaz, pour un gaz stérile destiné à constituer l'atmosphère interne de ladite enceinte.

Comme, selon une caractéristique avantageuse de l'invention, l'enceinte est en matière plastique, elle présente un coût réduit et peut être jetée après une seule utilisation.

D'autres caractéristiques et avantages de l'invention ressortiront de la description qui va suivre se rapportant à des formes de réalisation préférentielles, données à titre d'exemples non limitatifs, et représentées schématiquement sur les dessins ci-joints dans lesquels :

    - la fig. 1 est une représentation schématique, en perspective, d'une partie, formant carrousel, de l'appareillage selon l'invention,

    - la fig. 2 est une représentation schématique, en perspective, de l'appareillage selon l'invention tel qu'il se présente extérieurement, les éléments montrés sur la figure 1 n'étant pas représentés pour ne pas surcharger le dessin,

    - la fig. 3 est une représentation schématique, partiellement coupée, d'un appareillage selon l'invention dans une version simplifiée ne comportant pas de quai de triage,

    - la fig. 4 est une vue schématique en coupe de deux enceintes de fécondation selon l'invention, de leur montage sur le carrousel, et de leur branchement à des moyens d'alimentation en gaz.

L'appareillage de l'invention fait appel, de manière connue, à une aiguille de ponction folliculaire reliée par deux tuyaux respectifs à un flacon contenant un milieu de lavage et à un ajutage d'entrée dans un récipient tel qu'une enceinte qui sera décrite plus loin, un autre tuyau étant branché à un ajutage de sortie de ce récipient servant à le relier à une pompe. Cette partie de l'appareillage est donc conforme à l'état de la technique qui a été exposé plus haut, à l'exception du fait que le tube à essais de la technique antérieure peut être remplacé (mais ce n'est pas obligatoire) par l'enceinte déjà mentionée ; la partie en question de l'appareillage n'est pas représentée sur les dessins.

Pendant la récolte des ovocytes sur la "donneuse", on peut maintenir le tube ou l'enceinte de fécondation dans une mallette thermostatée en utilisant des tuyaux suffisamment longs. A la fin de la récolte, il suffit de clamper les tuyaux pour pouvoir transporter les ovocytes entre la clinique et le laboratoire d'examen dans cette mallette.

Au laboratoire, on ouvre la mallette, on prend une enceinte, que l'on place sur un support se trouvant dans une hotte à flux laminaire, ou même sous une couveuse, on ouvre le couvercle, puis on procède, à l'aide d'une loupe binoculaire, à la recherche et à l'examen des ovocytes, et on met les bons ovocytes dans le bassin de triage prévu dans les enceintes de récupération d'ovocytes, en vue de leur lavage dans un milieu approprié (ou dans un tube à essais, selon la technique connue).

Quand les ovocytes sont lavés, ils sont transvasés chacun dans un bassin identifié d'une seconde enceinte selon l'invention, en vue de leur fécondation in-vitro en présence de semence filtrée.

Toujours selon une caractéristique essentielle de l'invention, l'appareillage comporte un dispositif mobile, ici sous la forme d'un carrousel 1 recevant une multiplicité d'enceintes de fécondation 100, auquel est associé un dispositif optique de grossissement 2 pour examiner successivement le contenu des bassins des enceintes disposées sur le carrousel, des moyens d'éclairage (diascopique ou épiscopique) du contenu des enceintes (non représentés sur les dessins), des moyens d'alimentation 4 des enceintes disposées sur le carrousel en atmosphère gazeuse (cette atmosphère gazeuse introduite à l'intérieur des enceintes étant ici constituée par un mélange d'azote pour 90 %, d'oxygène pour 5 % et de gaz carbonique pour 5 %), et enfin des moyens de chauffage à une température constante (également non représentés) à savoir 37°C ± 0,1°C à l'intérieur d'une couveuse aménagée spécialement à cet effet, ainsi qu'éventuellement une ou plusieurs platesformes 5 de triage et de manipulation des ovocytes.

Le carrousel comporte un plateau 11 en forme de couronne circulaire, ou plusieurs plateaux en forme de couronnes circulaires disposés concentriquement, chaque plateau étant monté mobile en rotation sous l'action de moyens mécaniques ou moteurs électriques munis d'une roue d'entraînement caoutchoutée associée à un dispositif à crémaillère ou une poulie ou un galet d'entraînement, ce plateau comportant des emplacements de positionnement destinés chacun à recevoir une enceinte 100, par exemple des logements 12 présentant un contour semblable à celui des enceintes mais légèrement plus grand ou/et un nez de positionnement 46 en saillie au-dessus de la surface du plateau 11 et servant également à amener le gaz dans les enceintes comme cela sera décrit plus loin en détails. Le dispositif optique de grossissement 2 est une loupe binoculaire ayant un grossissement de 20 à 100 par exemple, dont l'axe optique est susceptible d'être amené au-dessus de chacun des logements 12 du carrousel 1.

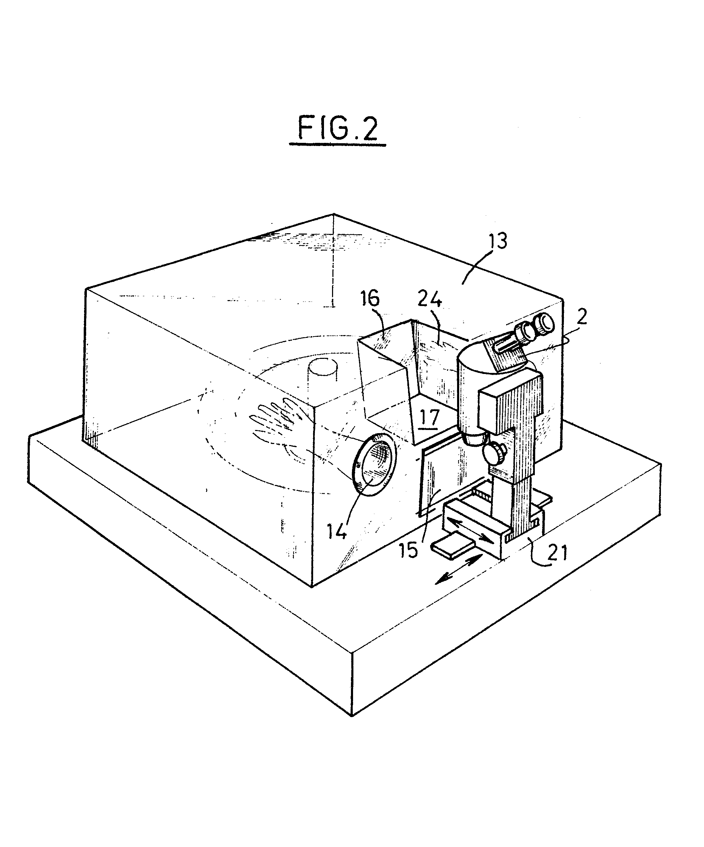

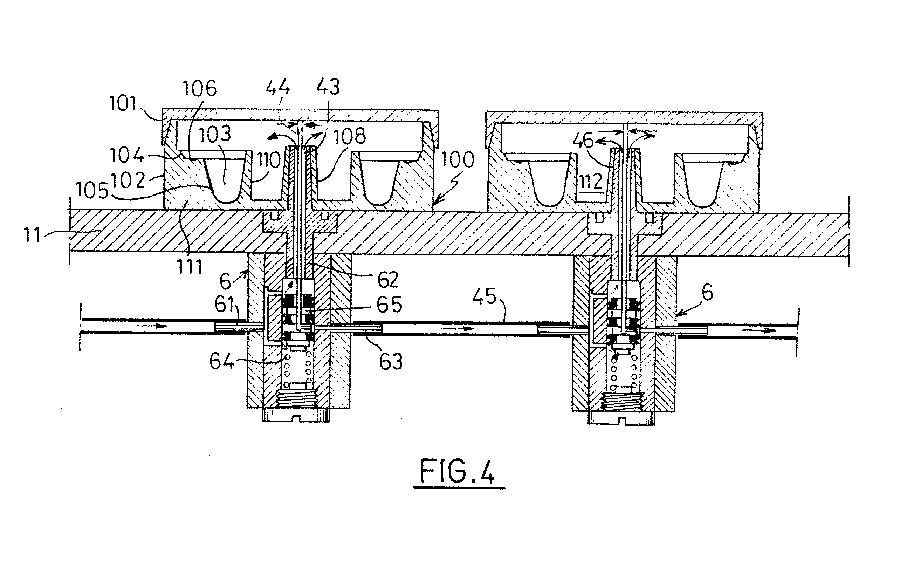

Cette loupe binoculaire, dont la lentille frontale est placée quelques centimètres (par exemple une douzaine de centimètres) au-dessus du haut de l'enceinte dont le contenu est à observer, peut être par exemple supportée par un bras solidarisé à un bâti disposé sensiblement au milieu du carrousel, et une articulation à genouillère peut être prévue, si l'axe optique de la loupe doit être déplacé pour observer plusieurs ovocytes ou embryons disposés dans des bassins séparés d'une même enceinte ou pour observer plusieurs enceintes disposées sur des plateaux différents. En fait, sur les dessins, c'est une autre forme de réalisation qui a été représentée, forme de réalisation dans laquelle le socle 21 de la loupe binoculaire comporte des glissières longitudinales et des glissières transversales permettant le mouvement de la loupe dans ces deux directions perpendiculaires tout en permettant le dégagement complet de la loupe, par rapport au carrousel, comme représenté sur les figures 2 et 3. Une autre possibilité consiste à prévoir un pont surmontant le carrousel et supportant la loupe binoculaire, mobile dans deux directions différentes. Le bon positionnement du plateau lors de sa rotation peut être assuré par des galets disposés à sa périphérie, et la rotation du plateau peut être obtenue en prévoyant que l'un de ces galets soit moteur. On peut également prévoir que le carrousel présente une mobilité dans son plan en plus de sa mobilité en rotation, par exemple au moyen d'une articulation centrale, et que la loupe binoculaire soit fixe. Cependant, selon une variante de réalisation préférentielle, les enceintes 100, circulaires, peuvent être tournées sur elles-mêmes en vue d'amener leurs bassins successivement en regard de l'optique qui dans ce cas pourrait être fixe ; on notera à cet égard que la configuration la mieux adaptée à se contenter d'une mobilité en rotation seulement du carrousel et à une fixité de la loupe binoculaire, est celle où l'examen des bassins est effectué dans une position de l'enceinte où les bassins sont situés sur la trajectoire circulaire des axes de rotation des enceintes autour de l'axe central du carrousel ; en effet, dans ce cas, une faible rotation du plateau 11 et une faible rotation de l'enceinte 100 se font selon deux petits arcs de cercle assimilables en première approximation à deux segments de droites perpendiculaires, ce qui donne une possibilité de positionnement parfait du bassin sous la loupe binoculaire, même si celle-ci est fixe.

La mise au point peut être faite ou réglée par des organes de commande de réglage grossier et de réglage fin, extérieurs ou intérieurs à la couveuse thermostatée, selon que toute ou partie de la loupe binoculaire est à l'extérieur.

Afin que les enceintes 100, et plus précisément leur contenu, puissent être examinés, sans que les enceintes soient ouvertes, au moyen du dispositif optique de grossissement 2, placé au-dessus d'elles, leur couvercle 101 est transparent. Si des moyens d'éclairage sont prévus en dessous des enceintes et/ou latéralement à celles-ci, il est nécessaire que le corps 102 lui-même des enceintes soit également transparent, et que les plateaux soient ou totalement transparents ou qu'ils le soient seulement aux endroits des éventuels logements 12 des enceintes, ou encore que ces logements comportent des ouvertures pour le passage du flux lumineux. Si la conception des enceintes est telle qu'elles puissent être tournées sur elles-mêmes, il suffit d'une seule ouverture par enceinte dans le plateau, ou que les enceintes soient disposées en porte à faux sur celui-ci, de telle sorte qu'en leur faisant subir une rotation sur elles-mêmes, on puisse amener successivement tous leurs bassins dans le ou les faisceau(x) lumineux émis par les moyens d'éclairage (non représentés) en direction du ou des bassin(s) non masqué(s) par le plateau.

Pour ne pas provoquer une élévation de température du contenu des bassins, le flux lumineux ou les flux lumineux concourants (par exemple deux), émanent d'une source de lumière froide par l'intermédiaire d'une optique de fibres.

Pour que le carrousel puisse être thermostaté et éventuellement que la pression puisse y être maintenue à une valeur désirée, la couveuse est équipée d'un capot transparent et étanche 13. Ce capot est muni de gants de manipulation 14 fixés à sa face avant ; cette face avant comporte également une fenêtre 15 permettant de travailler sous le capot 13 sans l'intermédiaire des gants 14 si nécessaire. Pour que l'objectif de la loupe puisse être amené à une distance correcte au-dessus des enceintes de fécondation, le capot comporte égale ment une échancrure 16 permettant d'amener la loupe binoculaire juste au-dessus de l'enceinte à examiner. L'examen des enceintes est fait à travers une glace interchangeable 17 montée sur glissières, une glace étant moins sujette aux rayures que le capot 13 en matière plastique , ou par l'intermédiaire d'un manchon, pour avoir une lecture directe; le retrait de la glace 17 permet d'ailleurs un accès supplémentaire à l'intérieur du capot. Il est ainsi possible de manipuler sous le capot (par exemple de dénuder les ovocytes) soit en se servant des gants 14, soit directement par la fenêtre 15 de la face avant, soit par l'échancrure 16.

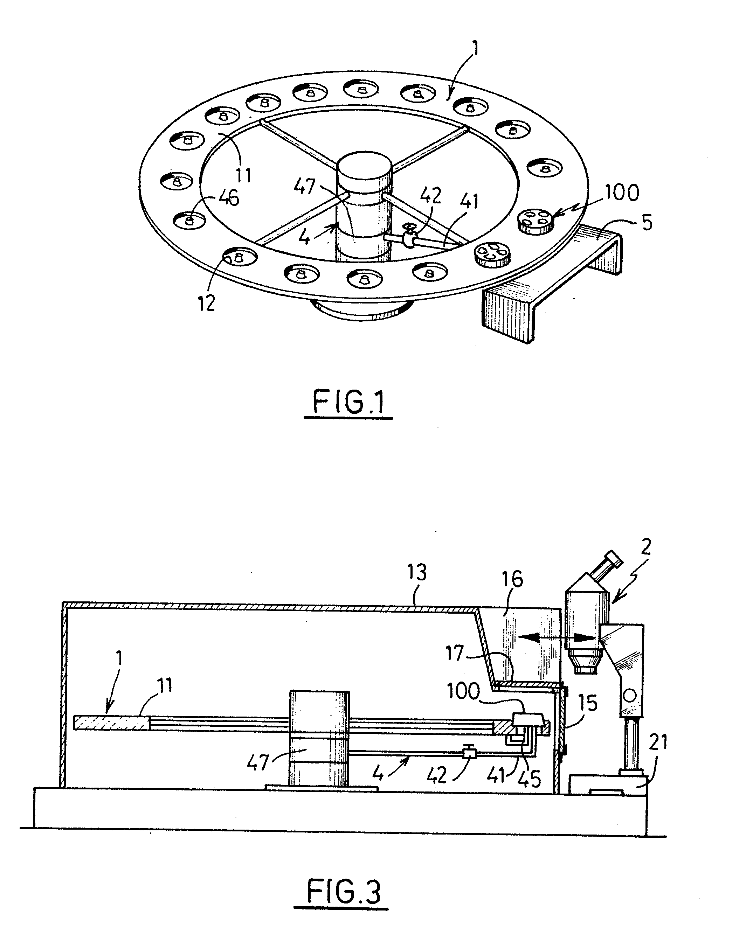

Selon une caractéristique préférentielle de l'invention, la couveuse peut être munie, comme c'est le cas sur la figure 1, d'une (ou plusieurs) plate(s)-forme(s) de triage et de manipulation, par exemple d'une plate-forme 5 mobile, extérieure au plateau 11. Ces plates-formes, dont la configuration doit être adaptée à ne pas entraver la libre rotation du plateau, sont disposées dans la couveuse et devant la loupe binoculaire, et reçoivent des supports magnétiques sur lesquels sont fixés des micromanipulateurs servant à dénuder les ovocytes et actionnés par l'opérateur de préférence au moyen des gants de manipulation 14.

L'alimentation des enceintes 100 en gaz carbonique est effectuée au moyen d'un conduit d'arrivée entrant centralement dans le carrousel et alimentant, par l'intermédiaire d'un joint tournant 47, une ou plusieurs conduites 41 rayonnant vers les plateaux en couronne ; chacune des conduites 41, qui peut comporter un robinet de commande 42, à moins qu'il existe un robinet de commande général, dessert plusieurs enceintes voisines, par exemple une dizaine, en série (on est limité par les pertes de charge) ; en conséquence, chaque conduite 41 débouche dans un premier emplacement de positionnement pour une enceinte par l'intermédiaire du nez 46, tronconique, déjà mentionné, comportant une relativement courte buse d'entrée 43 presque centrale ou parfaitement centrale, selon les formes de réalisation, dépassant très légèrement au-dessus du niveau supérieur des bassins des enceintes de fécondation ; le même logement comporte une buse de sortie 44 nettement plus haute pour être certain du total remplissage de l'enceinte par le gaz, selon les formes de réalisation, environ 1 millimètre en dessous du couvercle de l'enceinte, ou en butée contre celui-ci, quand il est en position de fermeture de l'enceinte, comme cela sera décrit plus en détails.

Cette buse de sortie est symétrique de la buse d'entrée par rapport au centre du logement ou coaxiale intérieurement à celle-ci. Elle est reliée à sa base, par un conduit intermédiaire 45 par exemple en caoutchouc, à la courte buse d'entrée de l'emplacement suivant, lequel comporte une buse haute de sortie reliée par un autre conduit intermédiaire à la buse d'entrée d'un autre emplacement, et ainsi de suite, jusqu'au dernier emplacement de la série, ce qui permet de chasser des enceintes de fécondation de cette série, l'air susceptible de contaminer leur contenu, et de le remplacer par le mélange de gaz stérile destiné à maintenir une atmosphère favorable à la fécondation. On comprendra donc que la courte buse d'entrée 43 et la buse haute de sortie 44 de chaque logement sont de préférence entourées d'un nez métallique central et tronconique 46, commun, pouvant être escamotable, dont l'orifice supérieur affleure sensiblement le niveau haut de la courte buse d'entrée si les buses sont côte à côte, et que la courte buse d'entrée est constituée par la paroi extérieure elle-même du nez 46 si la structure est coaxiale.

Les moyens de chauffage du meuble dans lequel sont placés le carrousel et tout le matériel associé pour l'examen des enceintes maintiennent celles-ci à la température désirée, de préférence au moyen d'un bain-marie (grâce à l'inertie thermique du liquide du bain, par exemple de l'eau) ; le liquide du bain peut être contenu soit dans les enceintes 100, soit éventuellement dans un bac disposé dans le carrousel lui-même et dans lequel les enceintes baignent en permanence si l'éclairage du contenu des bassins est épiscopique. Dans le cas d'ovocytes et d'embryons humains, la température est maintenue à 37°C ± 0,1°C. Les moyens moteurs actionnent les plateaux en rotation, pas à pas, au moyen du dispositif cranté permettant d'obtenir une course donnée à chaque pas de progression en rotation du plateau, sous commande automatique, ou à main ou à pied.

La couveuse peut être complétée par un tunnel destiné à protéger les enceintes contre les agressions de la lumière, notamment dans la gamme des rayons ultra-violets, ou encore, le capot de la couveuse peut être au moins partiellement en un matériau plus opaque dans la gamme des longueurs d'ondes indésirables.

Les boîtes de Pétri connues sont transparentes (corps et couvercle) et comportent en général plusieurs bassins où sont disposés les ovocytes à observer. Dans la mesure où les bassins sont de forme cylindrique, ils ne présentent qu'un intérêt très limité pour la présente application, car si les ovocytes et spermatozoïdes ou embryons se réfugient dans la région périphérique du fond, ils sont difficiles à observer et les embryons sont très difficiles à prélever en vue de leur transfert final.

Aussi, selon l'invention, les enceintes 100 destinées à l'installation qui vient d'être décrite, sont non seulement transparentes, ou au moins translucides en ce qui concerne leur corps, mais présentent un ou plusieurs bassins latéraux 103 ayant une forme particulière (un seul bassin étant suffisant pour les enceintes de récolte).

Ces bassins 103 ont des parois intérieures dont la partie supérieure 104, circulaire, est de préférence inclinée et converge vers le bas (de manière légèrement conique rectiligne ou incurvée) ; la partie 105 du fond, ici excentrée, de préférence converge également vers le bas, et le fond proprement dit est incurvé avec une convexité tournée vers le bas (il est par exemple en forme de calotte sphérique ou hémisphérique) ; la partie supérieure et la partie inférieure sont séparées, sur au moins une partie du pourtour du bassin, par une zone intermédiaire plane et horizontale 106 comportant un petit alvéole circulaire adapté à recevoir la goutte de milieu contenant l'ovoctye. L'inclinaison de la partie supérieure 104 des parois permet d'introduire, dans les bassins 103, des instruments dans une position autre que la position verticale, qui n'est pas toujours la plus pratique ; la partie intermédiaire plane et horizontale 106 sert à dénuder l'ovocyte contenu dans la goutte disposée dans l'alvéole, au moyen d'un micro-manipulateur, et la forme convergente et arrondie de la partie inférieure 105 des bassins permet un confinement central de l'ovocyte et des spermatozoïdes et facilite donc d'une part leur rencontre et d'autre part la recherche de l'ovocyte, et ultérieurement de l'embryon, au moyen de la loupe binoculaire, puisqu'il reste facilement repérable par l'objectif de celle-ci.

De plus, les enceintes 100 selon l'invention présentent une bonne étanchéité en vue de supprimer les risques de pollution et de fuite de gaz ; cette étanchéité est obtenue au moyen de la coopération de parois du corps 102 et de parois correspondantes du couvercle 101 qui sont inclinées au moins sur une partie de leur hauteur et de même angle d'inclinaison, ce qui permet un bouchage bien hermétique par coincement des zones se faisant vis à vis. En variante, l'étanchéité peut être assurée par des pas de vis complémentaires prévus recpectivement sur le corps et le couvercle des enceintes, en vue d'assurer leur fermeture. Dans le cas où l'enceinte comporte plusieurs bassins 103, il est nécessaire de repérer ceux-ci, ou plutôt leur contenu ; à cet effet, le corps ou le couvercle comporte un ou plusieurs repères d'identification ;

ainsi, les bassins peuvent être affectés d'un numéro d'identification, et il en est de même de chaque enceinte ; si l'enceinte présente une symétrie, le corps et/ou le couvercle sont munis d'un repère de positionnement de telle sorte que l'indication éventuelle donnée par le couvercle ne soit pas erronée à cause d'un mauvais positionnement de celui-ci. La base du corps 102 des enceintes peut comporter une semelle légèrement débordante (non représentée sur les dessins), destinée à s'encliqueter avec des organes de retenue prévus dans leur logement, en plusieurs endroits de leur pourtour, de manière à éviter qu'une tentative de retrait de leur couvercle, (lequel retrait peut être un peu "dur" à cause du coincement conique du couvercle sur le corps), se solde par un soulèvement du corps lui-même avec toutes les conséquences fâcheuses qui peuvent en résulter pour le contenu des bassins. Dans le cas où les buses d'entrée et de sortie de gaz sont éloignées, les enceintes comportent dans leur fond un ajutage d'entrée et un ajutage de sortie (ou trop-plein) dans lesquels sont destinées à s'ajuster respectivement ces buses d'entrée et de sortie, de telle sorte que le gaz stérile constitue l'atmosphère interne de l'enceinte, notamment pour en fixer le pH, emplisse l'intérieur des bassins en particulier en contact avec le milieu où se trouve l'ovocyte et les spermatozoïdes, ou l'embryon, y constitue un matelas de gaz, chasse l'air résiduel, et que le superflu s'échappe par le trop-plein. Dans la forme de réalisation représentée sur les dessins où les buses 43, 44 sont coaxiales, ou dans le cas où elles sont quasiment centrales, et entourées du nez tronconique commun 46, les enceintes sont elles-mêmes munies, centralement à leur base, d'un tronc de cône d'étanchéité 108, tronconique intérieurement et extérieurement, dont la surface interne est adaptée à s'emboîter sur le tronc de cône du nez 46; ce tronc de cône d'étanchéité 108 est ouvert à son extrémité supérieure qui constitue donc un ajutage unique pour laisser le passage au gaz entrant dans l'enceinte par la buse d'entrée 43, et à la buse de sortie 44 ; cette extrémité supérieure du tronc de cône d'étanchéité 108 dépasse légèrement au-dessus du nez tronconique 46 du logement de l'enceinte, et du niveau supérieur des bassins. Dans une telle forme de réalisation, au lieu d'être conçus sous la forme d'une semelle coopérant avec des organes de retenue prévus sur le plateau 11, les moyens de retenue des enceintes 100 sur le plateau 11 peuvent être constitués par des organes susceptibles de s'accoupler, respectivement sur le nez et le tronc de cône d'étanchéité 108 de l'enceinte. Dans le présent exemple, les bassins sont dimensionnés pour recevoir environ un demi-centimètre cube de milieu liquide de fécondation (la capacité totale du bassin étant de l'ordre du centimètre cube). Grâce aux caractéristiques qui viennent d'être énumérées, les enceintes peuvent donc avantageusement servir de nid de fécondation pour les ovocytes. De plus, il est possible de contrôler sur place la santé des ovocytes et des spermatozoïdes, et la mise en culture des embryons, évitant ainsi les manipulations superflues. Il faut noter que dans la mesure où les enceintes sont en matière plastique, (matériau relativement bon marché même lorsqu'il doit être, comme ici, neutre et de qualité alimentaire), il est possible de ne s'en servir qu'une seule fois, puis de les jeter après usage.

On a déjà vu que dans certains cas, il peut être nécessaire de disposer d'une loupe binoculaire 2 mobile, (grâce à l'articulation du type genouillère déjà mentionnée ou à un montage sur glissières déjà envisagé) pour amener son axe optique dans le prolongement de l'axe central de chaque bassin 103, successivement, ou encore d'un carrousel mobile ; en revanche, si les enceintes sont agencées pour pouvoir tourner sur elles-mêmes, on peut éventuellement se passer d'une mobilité de la loupe binoculaire, ou du carrousel, la rotation de l'enceinte sur son emplacement étant possible en passant la main à travers une fenêtre du capot ou dans un gant, à condition que les entrées et sortie de gaz soient agencées comme cela a été décrit en détail plus haut.

Par ailleurs, il est nécessaire que le couvercle 101 d'une enceinte quelconque puisse être ôté sans que soit interrompue l'alimentation en gaz des autres enceintes du fait de l'échappement de celui-ci dans l'atmosphère.

A cet effet, il peut être prévu par exemple un capuchon amovible, en métal ou en matière plastique, présentant une cavité interne en tronc de cône, complémentaire de la surface extérieure du tronc de cône d'étanchéité 108 faisant partie de la base de l'enceinte. Ce capuchon n'est normalement pas en place sur le tronc de cône d'étanchéité 108 de telle sorte que le gaz puisse pénétrer dans l'enceinte, mais il doit être emboîté légèrement à force sur ledit tronc de cône d'étanchéité dès l'ouverture de l'enceinte ; il est de préférence suffisamment haut pour que l'on soit obligé de le retirer avant de remettre le couvercle de celle-ci, pour être certain de réintroduire alors du gaz à l'intérieur ; de ce fait, le fond du capuchon (en haut de celui-ci) laisse un léger espace au-dessus de la buse haute 44 de sortie du logement de l'enceinte, nécessaire pour le passage du gaz provenant de la buse d'entrée 43, afin que ce gaz puisse continuer à alimenter les enceintes suivantes de la série.

Une autre solution, également non représentée, fait appel à des vannes disposées dans la conduite, d'arrivée 41 et dans les conduits 45 ; chacune de ces vannes comporte une sortie reliée à la buse d'entrée de l'enceinte immédiatement en aval et à l'entrée de la vanne de l'enceinte suivante. La base de chaque enceinte comporte une encouche susceptible de coopérer avec une bille logée dans le plateau 11, laquelle bille est en relation avec un axe horizontal commandant une biellette verticale d'actionnement de la vanne ; de la sorte, lorsque la bille est dans l'encoche, la biellette est libérée et permet le passage du gaz dans la direction choisie. Ainsi, dans une première position, l'enceinte directement en aval de la vanne est alimentée en gaz, et dans une seconde postiion, le mouvement de la bille inverse la position de la vanne et c'est l'enceinte suivant celle qui est directement en aval de ladite vanne qui est alimentée, à moins que cette enceinte ne soit elle-même tournée de manière à ne pas pas être alimentée, auquel cas le processus se répète, jusqu'à la fin de la série d'enceintes.

Dans la forme de réalisation représentée sur la figure 4, à chaque emplacement du plateau tournant 11 prévu pour une enceinte, est adapté un distributeur de gaz 6 à une entrée 61 et deux sorties 62, 63 ; ce distributeur présente une structure cylindrique dont l'axe longitudinal est dans le prolongement de l'axe longitudinal du nez 46 ; l'entrée 61 de chaque distributeur est reliée soit à la conduite 41 pour la première enceinte de la série, soit au conduit 45, amont, en série ; les deux sorties 62, 63 du distributeur sont reliées respectivement à la buse d'entrée de l'enceinte immédiatement en aval (qui se trouve juste au-dessus du distributeur) et au conduit d'alimentation 45 de la suivante ; dans cette forme de réalisation, la buse de sortie 44 et la buse d'entrée 43 sont à structure coaxiale, la buse de sortie 44 étant intérieure à la buse d'entrée 43 et étant mobile en translation longitudinale, une force de rappel étant exercée par un ressort 64 pour tendre à la ramener en position haute par l'intermédiaire du piston 65 du distributeur 6 ; de plus, cette buse de sortie 44 comporte des trous périphériques à sa partie supérieure, comme on l'a vu afin d'être certain que l'enceinte est emplie de gaz quand elle est fermée. Lorsque le couvercle 101 est placé sur son enceinte, il main tient la buse de sortie 44 en position basse ; le gaz est alors transmis à la buse d'entrée 43 de cette enceinte par le distributeur disposé sous celle-ci, il emplit l'enceinte, et repart par les trous périphériques de la buse de sortie 44 ; le piston 65 du distributeur 6 étant maintenu vers le bas, le surplus de gaz part alimenter le distributeur de l'enceinte suivante. Lorsque le couvercle 101 d'une enceinte est enlevé, le ressort 64 pousse le piston 65 vers le haut, du fait que la buse de sortie 44 remonte, l'arrivée de gaz dans l'enceinte de fécondation est donc coupée, et le gaz passe dans la partie inférieure du distributeur 6 pour alimenter directement le distributeur 6 suivant. Ces fonctions sont assurées au moyen de trois joints d'étanchéité à double lèvres prévus dans des logements creusés à la périphérie du piston, déterminant les voies de passage possibles pour le gaz.

La présence des distributeurs garantit qu'en cas d'oubli de la fermeture du robinet de gaz général, même si des enceintes sont démunies de leur couvercle, le gaz ne peut s'échapper.

Entre le tronc de cône d'étanchéité 108 de la base de l'enceinte, les protubérances 110 qui supportent les bassins, et les parois extérieures 111 de l'enceinte, est délimitée une cavité 112 destinée à recevoir un liquide (sérum stérile ou eau distillée) permettant l'humidification de la boîte, et éventuellement, si nécessaire, un barbotage du gaz, par exemple pour réchauffer celui-ci à la température la plus favorable.

Ces enceintes peuvent être utilisées :
- dans les actuelles cloches à incubation, les tuyaux d'admission de gaz restant alors fermés et le couvercle seulement posé, non hermétiquement (a la place de boîtes de Nunc ou autres),
- avec support unique dans les couveuses (à la place des tubes),
- en carrousel.

En variante, les enceintes peuvent comporter un orifice supplémentaire pour y placer une sonde de contrôle de la température et/ou du taux d'humidification pendant la fécondation, par exemple par le nez central.

La mise en oeuvre de l'appareillage selon l'invention s'effectue selon le processus suivant :

On prélève tout d'abord de manière connue, par ponction folliculaire, les ovocytes au moyen d'une aiguille, on identifie (sous hotte à flux laminaire ou sous couveuse) les ovocytes aptes à être fécondés dans de bonnes conditions, on transfère chaque ovocyte dans un bassin 103 identifié respectif d'une enceinte de fécondation 100, on introduit dans les bassins un milieu liquide adapté à la fécondation, on place des spermatozoïdes préalablement filtrés donc vivants dans les bassins, on ferme de manière étanche le couvercle de l'enceinte de fécondation, on ouvre le robinet d'arrivée du gaz stérile (dont la composition a été donnée plus haut) réchauffé pour éviter les chocs thermiques, et on introduit dans l'enceinte (éventuellement avec un barbotage) ce gaz stérile en chassant l'air résiduel, et après écoulement d'un certain temps, sans ouvrir l'enceinte, on examine successivement le contenu des bassins au moyen de la loupe binoculaire.

Comme cela a déjà été mentionné, la ponction folliculaire peut mettre en oeuvre directement des enceintes selon une forme appropriée de l'invention, placées en mallette étanche et thermostatée à 37° C ± 0,1° C, disposée horizontalement pendant la récolte des ovocytes ; les ovocytes sont alors récupérés dans la cavité centrale de l'enceinte, la ponction étant effectuée au moyen des ajutages d'entrée et de sortie de gaz (les ovocytes étant introduits par l'ajutage d'entrée et le pompage étant effectué par l'ajutage de sortie). Il est également possible de prévoir un filtre dans l'ajutage d'arrivée de gaz pour y piéger les ovocytes et il n'y a alors plus qu'à les en extraire.

Pour entrer plus en détailsdans la description du procédé selon l'invention, on peut signaler que lors de l'introduction du gaz stérile dans l'enceinte par la buse basse, ce gaz monte en pression et chasse l'air qui s'échappe par la buse haute ; avantageusement, un milieu tampon peut éventuellement avoir été préalablement injecté au moyen d'une seringue dans la cavité centrale 112 de l'enceinte, avec un niveau inférieur à celui du haut des bassins, ce qui peut permettre un barbotage du gaz introduit, par exemple en adaptant à la buse d'arrivée de gaz un tube mobile en U inversé plongeant dans le milieu tampon ; cette possibilité permet à l'opérateur d'obtenir une arrivée et un contrôle visuel de gaz parfaits grâce à l'observation des bulles ainsi formées dans le liquide ; elle permet également un réchauffage complémentaire du gaz. Ce barbotage entretient aussi une atmosphère humide constante et précise à l'intérieur de l'enceinte, qui est indispensable à la bonne conservation des ovocytes en les empêchant de se dessécher.

Afin de maintenir les enceintes et leur contenu à une température et dans des conditions sensiblement constantes, on les conserve dans la couveuse thermostatée à laquelle sont également associés des moyens d'humidification et de chauffage à température constante à 0,1°C près, par rapport à la température de l'espèce à l'état vivant, 37°C pour l'espèce humaine, par régulation par air pulsé.

Pour examiner le contenu des enceintes 100, on place les enceintes dans le carrousel 1, on entraîne le carrousel en rotation, et on fait défiler successivement, pas à pas, lesdites enceintes de fécondation sous la loupe binoculaire 2 sans risque de renverser ou contaminer le moindre ovocyte, et avec une grande rapidité et simplicité.

Lorsque les ovocytes d'une enceinte ont été fécondés par les spermatozoïdes, et que les embryons ont atteint le développement approprié, ces embryons sont, depuis le bassin, conditionnées en paillette en vue de leur congélation, ou dans une sonde de mise en place à partir desquels ils sont transférés dans l'utérus de la mère receveuse ; après cet usage unique, l'enceinte de fécondation peut être jetée.

Bien entendu, l'invention n'est pas limitée aux formes et aux modes de réalisation ci-dessus décrits et représentés, et on pourra prévoir d'autres formes ou d'autres conceptions sans sortir du cadre de l'invention.

**Revendications**

1. Appareillage de fécondation d'ovocytes in-vitro, du type comporenant des enceintes de fécondation comportant des bassins pour recevoir les ovocytes dans un milieu approprié ainsi que des spermatozoïdes en vue de la fécondation desdits ovocytes afin d'obtenir des embryons susceptibles d'être transférés dans une mère receveuse, lorsqu'ils ont atteint un développement approprié, appareillage caractérisé en ce qu'il comporte un dispositif mobile, tel qu'un carrousel (1), ayant au moins un plateau (11) mobile en rotation pas à pas et muni d'emplacements de positionnement (12) destinés à recevoir chacun une enceinte de fécondation (100), éventuellement un capot transparent de fermeture (13) entourant ledit dispositif mobile, un dispositif optique binoculaire de grossissement (2) pour examiner successivement le contenu des bassins, par le dessus de ceux-ci et du capot de fermeture éventuel, lequel est muni d'une fenêtre (17) en regard de l'optique de grossissement, et des moyens d'éclairage du contenu des bassins de fécondation.

2. Appareillage selon la revendication 1, caractérisé en ce qu'il comporte des moyens moteurs à roue caoutchoutée.

3. Appareillage selon la revendication 1, caractérisé en ce qu'il comporte des moyens d'alimentation (4) des enceintes (100) disposées sur le dispositif mobile (1), en atmosphère gazeuse.

4. Appareillage selon la revendication 3, caractérisé en ce que plusieurs enceintes (100) sont reliées en série aux moyens d'alimentation (4) en atmosphère gazeuse.

5. Appareillage selon la revendication 1, caractérisé en ce qu'il est logé au moins partiellement dans une couveuse comportant des moyens de climatisation à une température constante régulée.

6. Appareillage selon la revendication 1, caractérisé en ce qu'il comporte des moyens de déplacement relatif du carrousel et du dispositif optique binoculaire de grossissement, par exemple des moyens de déplacement à genouillère, à glissière ou à articulation.

7. Appareillage selon la revendication 1, caractérisé en ce qu'il comporte des moyens d'éclairage à lumière froide avec transmission par optique de fibres pour ne pas apporter de perturbation à la température de l'enceinte (100) en cours d'observation.

8. Appareillage selon la revendication 1, caractérisé en ce que le plateau (11) est au moins en partie muni de zones permettant la transmission de la lumière.

9. Appareillage selon la revendication 1, caractérisé en ce qu'il comporte une plate-forme (5) de triage et de manipulation à proximité du dispositif mobile (1).

10. Appareillage selon la revendication 1, caractérisé en ce que le dispositif mobile (1) comporte au moins un plateau (11) sur lequel les enceintes (100) sont disposées en porte à faux, leur partie en porte à faux étant dans le faisceau émis par au moins une optique de fibres.

11. Appareillage selon la revendication 1, caractérisé en ce que le dispositif mobile comporte, à chaque emplacement de positionnement pour une enceinte (100), un nez tronconique (46) comportant une buse d'entrée (43) et une buse de sortie (44) de gaz.

12. Appareillage selon la revendication 11, caractérisé en ce que la buse d'entrée (43) et la buse de sortie (44) sont disposées coaxialement.

13. Appareillage selon la revendication 12, caractérisé en ce que la buse de sortie (44) est mobile en translation longitudinale.

14. Appareillage selon la revendication 1, caractérisé en ce qu'il comporte, sous chaque emplacement de positionnement pour une enceinte, un organe de distribution de gaz adapté à diriger un gaz, destiné à constituer l'atmosphère des enceintes, vers cette enceinte ou un autre organe de distribution, selon que ladite enceinte est fermée ou non.

15. Procédé de fécondation d'ovocytes in-vitro dans lequel on prélève les ovocytes par ponction folliculaire au moyen d'une aiguille, procédé caractérisé en ce que l'on identifie les ovocytes aptes à être fécondés dans de bonnes conditions, on transfère chaque ovocyte dans un bassin (103) respectif d'une enceinte (100) de fécondation, on introduit dans les bassins un milieu liquide adapté à la fécondation, on place des spermatozoïdes, préalablement filtrés donc

**0 239 450**

vivants, dans les bassins identifiés, on ferme de manière étanche le couvercle de l'enceinte de fécondation, on ouvre alors une arrivée de gaz stérile et réchauffé éventuellement par barbotage pour éviter les chocs thermiques, et on introduit dans l'enceinte le gaz stérile qui chasse l'air résiduel, puis après écoulement d'un certain laps de temps, sans ouvrir l'enceinte, on examine le contenu des bassins au moyen d'un dispositif optique de grossissement (2).

16. Procédé selon la revendication 15, caractérisé en ce que l'on prélève les ovocytes par ponction folliculaire et on les introduit directement dans une enceinte comportant deux ajutages reliés respectivement à l'aiguille de ponction et à un dispositif de pompage, une cavité centrale; et des bassins de fécondation.

17. Procédé selon la revendication 15, caractérisé en ce que pour examiner le contenu des bassins (103), on place les enceintes (100) de fécondation dans un carrousel (1) que l'on entraîne en rotation, et on les fait défiler successivement pas à pas, sous une loupe binoculaire (2).

18. Enceinte de fécondation d'ovocytes pour appareillage de fécondation d'ovocytes in-vitro, caractérisée en ce qu'elle est étanche et au moins partiellement transparente et en ce qu'elle comporte des bassins (103) excentrés à paroi (104) inclinée convergeantvers le bas et présentant un fond excentré (105) incurvé à concavité tournée vers le bas et au moins une zone intermédiaire (106) plane et horizontale, ainsi qu'au moins un ajutage d'entrée et de sortie de gaz, pour un gaz stérile destiné à constituer l'atmosphère interne de ladite enceinte.

19. Enceinte de fécondation selon la revendication 14, caractérisée en ce qu'elle comporte un ajutage d'entrée de gaz et un ajutage de sortie de gaz de plus grande hauteur que l'ajutage d'entrée.

20. Enceinte de fécondation selon la revendication 18, caractérisée en ce qu'elle comporte un ajutage d'entrée et de sortie de gaz, destiné à recevoir une buse d'entrée de gaz (43) et une buse de sortie de gaz (44) de plus grande hauteur, entouré d'une paroi périphérique tronconique.

21. Enceinte de fécondation selon la revendication 20, caractérisée en ce qu'elle comporte un capuchon pour boucher l'ajutage lorsque l'enceinte est ouverte.

22. Enceinte de fécondation selon la revendication 18, caractérisée en ce qu'elle comporte une cavité (112) destinée à recevoir un milieu liquide tampon, stérile, pour permettre éventuellement un barbotage du gaz et constituer un bain-marie réduisant les chocs thermiques et assurant une bonne humidification de l'atmosphère gazeuse, et en ce que les bassins (103) sont creusés dans des régions en protubérance (110) par rapport à cette cavité (112).

23. Enceinte de fécondation selon la revendication 18, caractérisée en ce qu'elle comporte un corps (102) et un couvercle (101) ayant au moins des parties de parois inclinées en vue d'assurer un bouchage hermétique.

24. Enceinte de fécondation selon la revendication 18, caractérisée en ce qu'elle comporte un corps (102) et un couvercle (101), ainsi que des moyens d'encliquetage sur le plateau (11) pour empêcher tout mouvement du corps lors de son ouverture par retrait du couvercle.

25. Enceinte de fécondation selon la revendication 18, caractérisée en ce qu'elle est en matière plastique et à usage unique.

26. Enceinte de fécondation selon la revendication 18, caractérisée en ce que la zone intermédiaire (106) comporte un alvéole adapté à recevoir une goutte de liquide contenant un ovocyte en vue de dénuder ce dernier.

0239450

FIG.1

FIG.3

0239450

FIG.2

FIG.4

**Office européen des brevets**

**RAPPORT PARTIEL DE RECHERCHE EUROPEENNE**
qui selon la règle 45 de la Convention sur le brevet européen est considéré, aux fins de la procédure ultérieure, comme le rapport de recherche européenne

Numéro de la demande

EP 87 40 0428

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | TIME, MEDICINE, 10 septembre 1984, pages 34-40 C. WALLIS: The new origins of life" * Page 36, figures * | 1,5,9, 10 | A 61 D 7/02 C 12 M 3/00 A 61 B 17/42 A 01 K 67/02 |
| | --- | | |
| Y | GB-A- 980 681 (CRYOGENIC CO.) * Page 1, lignes 13-26; figures 1,3 * | 1,5,9, 10 | |
| | --- | | |
| A | DE-A-2 924 446 (P. HESSE) * Résumé; figure 2 * | 3,18 | |
| | --- | | |
| A | US-A-3 883 398 (ONO) * Figure 2 * | 23 | |
| | --- | | |
| A | US-A-4 321 330 (BAKER) * Figure 2 * | 22 | |
| | --- | | |

### RECHERCHE INCOMPLETE ./.

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes: 1-14,18-23

Revendications ayant fait l'objet de recherches incomplètes:

Revendications n'ayant pas fait l'objet de recherches: 15-17

Raison pour la limitation de la recherche:

Procédé essentiellement biologique d'obtention de végétaux ou d'animaux (voir art. 53 b) de la convention sur le brevet européen).

**DOMAINES TECHNIQUES RECHERCHES (Int. Cl.4)**

A 61 D
C 12 M
A 61 B
A 01 K

| Lieu de la recherche | Date d'achèvement de la recherche | Examinateur |
|---|---|---|
| La Haye | 18-06-1987 | EHRSAM |

Office européen
des brevets

**RAPPORT PARTIEL
DE RECHERCHE EUROPEENNE**

## DOCUMENTS CONSIDERES COMME PERTINENTS

| Catégorie | Citation du document avec indication, en cas de besoin, dés parties pertinentes | Revendica- tion concernée |
|---|---|---|
| A | FR-A-2 510 458 (N. ROHRER)<br>* En entier * | 7 |
| | ---------------- | |

**CLASSEMENT DE LA DEMANDE (Int. Cl.4)**

**DOMAINES TECHNIQUES RECHERCHES (Int. Cl.4)**

OEB Form 1505.3   06.78